# FASCICULE DE BREVET EUROPEEN

(11) **EP 2 046 301 B1**
(45) Date de publication et mention de la délivrance du brevet: **14.09.2016**
(21) Numéro de dépôt: 07823641.1
(22) Date de dépôt: 25.07.2007
(51) Int. Cl.: A61K 9/48, A61K 9/14, A61K 31/135, A61K 31/137, A61K 9/20

(54) **COMPOSITIONS PHARMACEUTIQUES DE SUBSTANCES ACTIVES DIFFICILEMENT DÉTOURNABLES DE LA VOIE D'ADMINISTRATION À LAQUELLE ELLES SONT DESTINÉES**
PHARMAZEUTISCHE ZUSAMMENSETZUNG VON WIRKSTOFFEN, DIE SCHWER VON IHREM DEFINIERTEN VERABREICHUNGSWEG ABZULENKEN SIND
PHARMACEUTICAL COMPOSITIONS OF ACTIVE SUBSTANCES DIFFICULT TO IMPROPERLY DIVERT FROM THEIR INTENDED ROUTE OF ADMINISTRATION

(30) Priorité: 28.07.2006 FR 0606956
(43) Date de publication de la demande: 15.04.2009
(73) Titulaire: Bouchara-Recordati, 92800 Puteaux (FR); Assistance Publique - Hôpitaux de Paris, 75004 Paris (FR)
(72) Inventeur: BERLEUR, Marie-Pierre, 92200 Neuilly Sur Seine (FR); CHAUMEIL, Jean-Claude, 91240 Saint Michel Sur Orge (FR); GUYON, François, 75005 Paris (FR); HOURI, Jean-Jacques, 94170 Le Perreux Sur Marne (FR); KIN, Thierry, 75018 Paris (FR); LE PALLEC, Gilles, 91700 Ste Genevieve Des Bois (FR); MOREAU, Elisabeth, 75007 Paris (FR)
(74) Mandataire: Cabinet Plasseraud
(86) Numéro de dépôt international: PCT/FR2007/051724
(87) Numéro de publication internationale: WO 2008/012474

(56) Documents cités:
- EP-A- 1 557 179
- WO-A-00/38649
- US-A- 4 070 494
- US-A1- 2004 126 428
- US-A1- 2006 110 327
- KIBBE A H (ED): "Cellulose, Microcrystalline" HANDBOOK OF PHARMACEUTICAL EXCIPIENTS, WASHINGTON, DC : APHA, US, 2000, pages 102-105, XP002217631 ISBN: 0-85369-381-1
- KIBBE A H (ED): "Cellulose, Powdered" HANDBOOK OF PHARMACEUTICAL EXCIPIENTS, WASHINGTON, DC : APHA, US, 2000, pages 102-105, XP002423964 ISBN: 0-85369-381-1

## Description

La présente invention concerne des compositions pharmaceutiques de substances actives, notamment classées comme stupéfiantes, difficilement détournables de la voie d'administration à laquelle elles sont destinées.

De nombreuses substances stupéfiantes sont utilisées en médecine humaine et notamment des opiacés tels que le lévacétylmétadol, la buprénorphine, l'hydromorphone, la méthadone, qui peuvent être utilisées dans le traitement de substitution aux opiacés ou dans le traitement de la douleur.

La biodisponibilité satisfaisante de la plupart de ces substances par voie orale a conduit au développement de diverses formes pharmaceutiques adaptées à cette voie d'administration.

Le caractère stupéfiant de ces substances peut conduire les sujets usagers de drogues à détourner la voie d'administration normale de ces médicaments, notamment pour les utiliser par injection parentérale et en particulier par voie intraveineuse. Pour cela, il peut suffire de mettre en solution la forme solide dans un faible volume d'eau.

D'autres substances, non stupéfiantes, pourraient être détournées de leur voie d'administration normale. Il s'agit par exemple de substances actives psychotropes. Les plus faciles à utiliser par les sujets usagers de drogues sont les gélules car les comprimés ont l'inconvénient de devoir être écrasés avant dissolution.

Il serait souhaitable de disposer de compositions pharmaceutiques solides sous forme de gélules (encore appelées capsules dures) de substances actives, notamment classées comme stupéfiantes, difficilement détournables de la voie d'administration à laquelle elles sont destinées.

Il serait également souhaitable que lesdites compositions pharmaceutiques solides conservent une bonne biodisponibilité.

WO 00/38649 par exemple décrit des compositions pharmaceutiques solides sous forme de comprimés qui après introduction dans une boisson de 20ml ou plus dont la composition produit soit des particules insolubles, inaptes à l'injection, soit la flottaison des comprimés.

US 2006/110327 décrit des formulations orales destinées à lutter contre le détournement d'usage d'un principe actif, comprenant un polymère formant un gel, un agent tensioactif irritant la muqueuse nasale et un agent provoquant un érythème du visage. Optionnellement, la composition peut contenir un agent désintégrant. EP 1 557 179 décrit une composition pharmaceutique solide comprenant un composé opiacé, un antagoniste des opiacés et un hydrocolloïde. WO 00/38649 décrit un comprimé pour administration par voie orale dotée de moyens visuels, comme la flottaison du comprimé ou la formation de particules insolubles, qui se manifestent après introduction du comprimé dans une boisson. US 4 070 494 décrit des compositions pharmaceutiques entériques destinées à lutter contre le détournement d'usage, incorporant un composé gélifiant. US 2004/126428 décrit des compositions pharmaceutiques sous forme de comprimés qui comprennent un noyau contenant un résinate d'opiacé et un enrobage. Le Handbook Of Pharmaceutical Excipients, Washington, Dc: Apha, US, 2000, décrit divers excipients.

Or après de longues recherches la demanderesse a mis au point une composition pharmaceutique solide sous forme de gélule, notamment pour administration orale, produisant un gel en présence d'eau, notamment une faible quantité d'eau, compatible avec une injection parentérale, donnant toute satisfaction.

C'est pourquoi la présente demande a pour objet une composition pharmaceutique solide sous forme de gélule contenant au moins
- un principe actif stupéfiant ou psychotrope ainsi que
- au moins un agent gélifiant choisi parmi l'hydroxyéthylcellulose, l'hydroxypropylcellulose, l'hydroxypropyl méthylcellulose, la méthylcellulose, l'éthyl méthylcellulose, la carboxyméthylcellulose sodique (CMC) et la carboxyméthylcellulose calcique et
- au moins un agent délitant dont le carboxyméthylamidon sodique,
l'agent gélifiant et l'agent délitant étant pondéralement présents dans un rapport de 0,40 à 0,60 /1 et la teneur pondérale en agent(s) gélifiant(s) étant de 10 à 20 % de la composition.

L'agent gélifiant et l'agent délitant sont pondéralement présents dans un rapport de l'un à l'autre de 0,40 à 0,60 / 1, notamment de 0,45 à 0,55 / 1, tout particulièrement de environ 0,5/1.

La composition pharmaceutique solide peut être toute forme connue de gélule, en gélatine ou autre matériau utilisé pour sa fabrication comme des éthers de cellulose hydrosolubles.

Dans la présente demande et dans ce qui suit, le terme "agent gélifiant" désigne un agent gélifiant ou augmentant la viscosité en solution aqueuse.

L'agent gélifiant est choisi parmi l'hydroxyéthylcellulose, l'hydroxypropylcellulose, l'hydroxypropyl méthylcellulose, la méthylcellulose, l'éthyl méthylcellulose, la carboxyméthylcellulose sodique (CMC), la carboxyméthylcellulose calcique, notamment la carboxyméthylcellulose calcique et la carboxyméthylcellulose sodique (CMC), particulièrement cette dernière. L'acide alginique ou ses sels peuvent aussi être utilisés.

La teneur pondérale en agent(s) gélifiant(s) de 10 à 20 %, sera de préférence de 10 à 15 %, notamment de 10 à 12 %, tout particulièrement de 10 % environ de la composition totale. L'homme de l'art pourra adapter celle-ci en fonction du choix de l'agent gélifiant. Elle ne dépassera généralement pas 20 % en poids, de préférence pas 15 % en poids, notamment pas 12 % en poids, particulièrement pas 10 % en poids de la composition totale.

Par exemple plusieurs grades de CMC sont disponibles dans le commerce. Ces CMC diffèrent entre elles par leurs caractéristiques chimiques et physiques. Une concentration de 4,0 à 6,0 % en CMC est généralement requise pour permettre la formation d'un gel en solution aqueuse. L'utilisation d'un grade de CMC avec un faible degré de substitution à une concentration de 1 % permet par exemple d'obtenir un gel de forte viscosité 2500 - 4500 mPa.s compatible avec le but recherché.

Dans la présente demande et dans ce qui suit, le terme " agent délitant" désigne un agent délitant ou dispersant.

Au moins un agent délitant est le carboxyméthylamidon sodique.

La teneur pondérale en agent(s) délitant (s) sera avantageusement de 20 à 40 %, de préférence de 20 à 30 %, tout particulièrement de 20 % de la composition totale.

Il est à noter que l'invention met en oeuvre avantageusement des proportions d'agent délitant, par exemple le carboxyméthylamidon sodique (CMA), plus importantes que l'usage habituel (concentration usuelle de 2 à 8 %). L'agent délitant permet une dispersion plus rapide du mélange de poudre et ainsi un mouillage des poudres, favorisant la gélification du mélange.

Dans des conditions préférentielles de mise en oeuvre de l'invention, la teneur en agent gélifiant et délitant est dosée pour permettre une gélification immédiate de la poudre d'une composition pharmaceutique solide ci-dessus après addition d'une solution aqueuse, de préférence d'eau, c'est-à-dire en moins de 1 minute, de préférence en moins de 30 secondes, particulièrement en moins de 20 secondes, tout particulièrement en moins de 15 secondes après début de l'agitation du mélange de la poudre et de la solution aqueuse. L'agitation est réalisée à l'aide d'un mouvement alternatif de retournement manuel d'un tube à hémolyse d'un volume de 5 ml renfermant un mélange de 150 mg de la poudre avec 3 ml d'eau, à la vitesse de 50 retournements à 180° du récipient par minute, à température ambiante. Le volume de 3 ml est en particulier le volume approximatif qu'utilisent les drogués pour s'injecter un stupéfiant présenté sous forme solide.

La composition pharmaceutique solide de l'invention pourra comprendre à titre de principe actif des composés organiques pharmacologiquement actifs, et de préférence des substances stupéfiantes utilisées en médecine notamment humaine. et particulièrement des opiacés tels que le lévacétylmétadol, la buprénorphine, l'hydromorphone ou la méthadone.

Les substances stupéfiantes sont notamment les substances actives susceptibles d'être utilisées en thérapeutique humaine et listées dans les Annexes I, II, II et IV de l'Arrêté du 22 février 1990 fixant la liste des substances classées comme stupéfiants (J.O. de la République Française du 07/06/1990).

On peut citer à titre de substances actives classées comme stupéfiantes et listées dans l'Annexe I par exemple la méthadone, l'hydromorphone, l'oxycodone, à titre de substances actives classées comme stupéfiants et listées dans l'Annexe II par exemple la codéine, le dextropropoxyphène, la dihydrocodéine, à titre de substances actives classées comme stupéfiants et listées dans l'Annexe III par exemple l'acide gamma-hydroxybutyrique, la méthaqualone, le méthylphénidate et à titre de substances actives classées comme stupéfiants et listées dans l'Annexe IV le nabilone et ses sels, les tétrahydrocannabinols et leurs sels.

Les substances non stupéfiantes sont par exemple les substances actives classées comme psychotropes susceptibles d'être utilisées en thérapeutique humaine et listées principalement dans les tableaux III (présentant une valeur thérapeutique moyenne à grande selon la convention de Vienne) et IV (présentant une valeur thérapeutique faible à grande selon la convention de Vienne) selon l'Arrêté du 22 février 1990 fixant en France la liste des substances classées comme psychotropes.

On peut citer à titre de substances actives classées comme psychotropes et listées principalement dans le tableau III par exemple la buprénorphine et le flunitrazépam et à titre de substances actives classées comme psychotropes et listées principalement dans le tableaux IV des barbituriques comme par exemple, l'allobarbital, le barbital, le phénobarbital, des benzodiazépines comme par exemple, le diazépam, le lorazépam, le tétrazépam, des hypnotiques apparentés aux benzodiazépines comme par exemple le zolpidem, d'autres anxiolytiques comme par exemple, le méprobamate.

Il peut aussi s'agir, tant pour les substances stupéfiantes que pour les substances non stupéfiantes, de leurs isomères dans les cas où ils peuvent exister, conformément à la formule chimique correspondante desdites substances, des esters et éthers desdites substances ou desdits isomères, des différents sels desdites substances, de leurs isomères, de leurs esters et éthers.

Dans la composition de l'invention, compte tenu des posologies habituelles du principe actif utilisé, la teneur en principe actif pourra varier pondéralement dans l'intervalle avantageusement de 0,3 à 54 %, de préférence de 0,5 à 40 %, notamment de 0,5 à 30,0 %, tout particulièrement de 0,6 à 27,0 % de la composition totale. À titre d'exemple, la quantité d'une dose unitaire en principe actif pourra aller de 1 mg à 40 mg pour le chlorhydrate de méthadone.

Dans la composition de l'invention, le principe actif sera aussi généralement mélangé avec au moins un excipient inerte, souvent pondéralement majoritaire, intervenant comme agent diluant tel que par exemple, le phosphate de calcium, le lactose, le mannitol, la cellulose microcristalline, ou l'amidon.

La substance active sera aussi généralement mélangée avec au moins un agent lubrifiant favorisant l'écoulement de la poudre lors de la fabrication industrielle tel que par exemple, le talc, l'amidon de maïs, la silice colloïdale anhydre ou tout autre agent équivalent.

Le principe actif sera aussi généralement mélangé avec au moins un agent lubrifiant permettant de réduire les frictions inter particulaires tel que l'acide stéarique ou ses sels (stéarate de calcium, stéarate de magnésium), le lauryl sulfate de sodium, ou tout autre agent offrant des propriétés similaires.

La teneur pondérale en agent(s) lubrifiant (s) sera avantageusement de 0,25 à 5 % %, de préférence de 0,5 à 2,5 %, notamment de 0,75 à 1,5 %, tout particulièrement de 0,8 à 1,2 % de la composition totale.

Tout autre additif compatible avec la composition et les propriétés intrinsèques de la forme pharmaceutique tel que des anti-agglomérants, des antioxydants, des colorants, des vitamines, des sels minéraux, des agents de sapidité, des agents de lissage, de montage, d'isolation ou leurs mélanges peuvent être envisagés dans le développement de la composition ci-dessus.

Les excipients choisis dans la composition seront avantageusement inertes chimiquement vis à vis de la substance active pour permettre l'obtention d'une forme pharmaceutique stable dans le temps, dans les conditions normales de conservation.

Sur le plan physique, les excipients généralement majoritaires (agents diluant, gélifiant et délitant) présenteront de préférence un profil granulométrique adapté.

Par exemple, 90 % des particules auront une taille inférieure à environ 130 µm et 50 % des particules une taille supérieure à environ 50 µm pour permettre un mélange homogène avec le principe actif dont 90 % des particules sont inférieures à 380 µm avec une médiane de distribution située aux environs de 180 µm. (Taille des particules évaluée par la technique de diffraction laser)

Le rapport pondéral entre l'agent gélifiant et l'agent délitant est un aspect important. Le mélange en poudre de diverses proportions de constituants a été mis en contact avec divers volumes d'eau. Les observations du mélange de poudres après mise en contact avec l'eau et agitation au vortex pendant une durée de 5 secondes ont été : avant l'agitation, une dispersion du mélange de poudre ou l'apparition d'agglomérats après l'agitation, la formation d'un gel ou la solubilisation du mélange de poudres, le maintien d'agglomérats ou la persistance de poudre. Le rapport pondéral spécifique entre l'agent gélifiant et l'agent délitant permet d'obtenir un délitement rapide du mélange de poudre en présence d'eau, généralement sans agitation, et la formation d'un gel immédiatement après agitation.

La présente invention a également pour objet un procédé de préparation d'une composition pharmaceutique solide telle que définie ci-dessus, caractérisé en ce que l'on mélange les différents constituants et les place dans une gélule pour obtenir la composition attendue.

Les gélules objet de la présente invention possèdent de très intéressantes propriétés et qualités. Si l'extraction de principes actifs de toute forme pharmaceutique reste toujours possible, les compositions pharmaceutiques solides ci-dessus la rendent complexe et longue et permettent d'éviter la pratique d'une injection facile et rapide par les dispositifs habituels qu'utilisent les usagers de drogue, après simple mise en solution du contenu de la gélule dans un faible volume d'eau.

Le rapport spécifique entre l'agent gélifiant et l'agent délitant permet d'obtenir un délitement rapide du mélange de poudre en présence d'eau, généralement sans agitation, et la formation d'un gel immédiatement après agitation.

La mise en contact de la poudre issue de la composition avec une solution aqueuse produit la formation d'un gel qui rend impossible ou au moins très difficile l'aspiration du mélange aqueux. Le critère retenu fait référence à la norme EN ISO 7886-1 (« Seringues hypodermiques stériles, non réutilisables-partie 1 : Seringue pour utilisation manuelle. Annexe G : Méthode d'essai des forces nécessaires pour faire fonctionner le piston - Tableau G1 : Valeurs proposées), les limites se situant entre 0,25 et 1 daN.

La viscosité élevée de la solution obtenue, en présence d'eau, en utilisant la composition objet du brevet ne diminue pas la biodisponibilité du principe actif.

Ces propriétés sont illustrées ci-après dans la partie expérimentale. Elles justifient l'utilisation des compositions ci-dessus décrites, dans la fabrication de gélules destinées à la voie orale.

La présente demande a aussi pour objet un procédé pour rendre difficilement injectable une composition pharmaceutique solide comprenant au moins un principe actif, caractérisée en ce que l'on prépare un mélange renfermant le principe actif ainsi qu'au moins un agent gélifiant et au moins un agent délitant, l'agent gélifiant et l'agent délitant étant pondéralement présents dans un rapport de 0,3 à 0,7 / 1.

La présente demande a également pour objet l'utilisation d'un mélange renfermant au moins un agent gélifiant et au moins un agent délitant, l'agent gélifiant et l'agent délitant étant pondéralement présents dans un rapport de 0,3 à 0,7 / 1 pour rendre difficilement injectable une composition pharmaceutique solide comprenant au moins un principe actif.

Les conditions préférentielles de mise en oeuvre des compositions pharmaceutiques solides ci-dessus décrites s'appliquent également aux autres objets de l'invention visés ci-dessus, notamment au procédé pour rendre difficilement injectable une composition pharmaceutique solide ci-dessus et à l'utilisation d'un mélange renfermant au moins un agent gélifiant et au moins un agent délitant, l'agent gélifiant et l'agent délitant étant pondéralement présents dans un rapport de 0,3 à 0,7 / 1 pour rendre difficilement injectable le contenu d'une gélule comprenant au moins un principe actif.

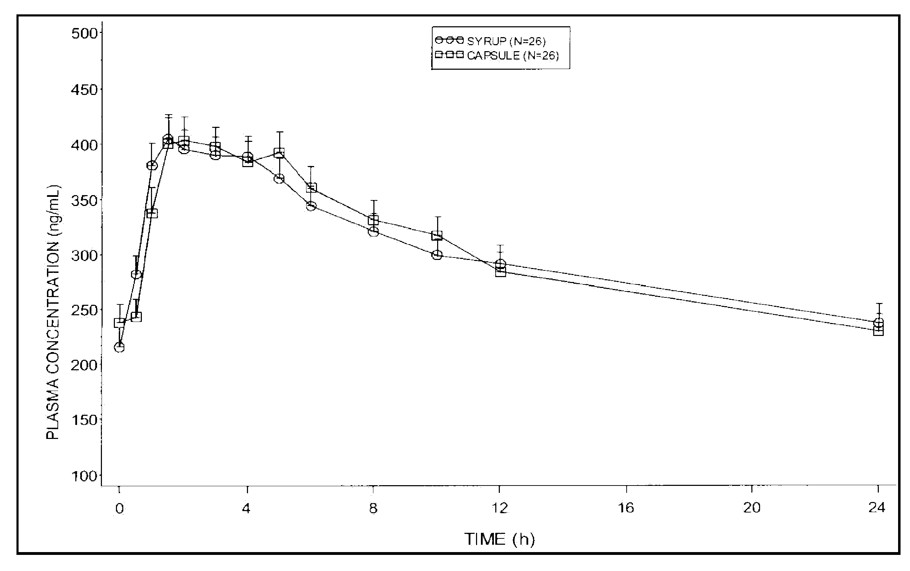

La figure 1 représente le profil plasmatique de la méthadone après administration répétée *in vivo,* chez l'homme, de 60 mg chlorhydrate de méthadone sous forme sirop (référence) et sous forme gélule (formule test) chez 26 patients. (Syrup = Sirop ; Time = Temps).

Les exemples qui suivent illustrent la présente demande. Ils ont été réalisés avec des produits stupéfiants pour lesquels la technique de l'invention présente le plus d'intérêt, mais d'autres principes actifs auraient pu être mis en oeuvre.

### Exemple 1 : Poudre

On a réalisé une poudre ayant la composition pondérale suivante :

| | % |
|---|---|
| Chlorhydrate de méthadone | 5,0 |
| Carmellose sodique | 10,0 |
| Carboxyméthylamidon sodique | 20,0 |
| Stéarate de magnésium | 1,0 |
| Silice colloïdale anhydre | 1,0 |
| Lactose monohydraté | 63,0 |

### Exemple 2 : Gélules de chlorhydrate de méthadone

On a réalisé des gélules de gélatine dure renfermant une poudre ayant la composition pondérale suivante :

| | % |
|---|---|
| Chlorhydrate de Méthadone | 20,0 |
| Carmellose sodique | 10,0 |
| Carboxyméthylamidon sodique | 20,0 |
| Stéarate de magnésium | 1,0 |
| Silice colloïdale anhydre | 1,0 |
| Lactose monohydraté | 48,0 |

Une gélule renferme 100 mg du mélange ci-dessus.

L'utilisation d'une quantité de carboxyméthylcellulose sodique supérieure à celle de la présente invention (> 10 %) entraîne une diminution de la teneur en eau de l'enveloppe de gélatine, la rendant cassante, ce qui diminue sa stabilité.

### Exemple 3 : Essai d'injection de principes actifs extraits d'une composition de l'exemple 2

La difficulté à injecter le médicament après sa mise en solution repose sur des essais de stabilité des gels, de viscosité et de passage par les aiguilles de seringues avec mesure de la force appliquée au piston.

La difficulté d'injection de la formule développée a été vérifiée en utilisant un mode opératoire adapté aux pratiques connues des consommateurs de drogues illicites pour mettre en solution le contenu des gélules, puis :
- en observant la difficulté d'aspiration, par une aiguille montée ou sertie de différents diamètres intérieurs,
- en mesurant les concentrations en substance active des solutions préparées à partir de différents dosages en vue de calculer les rendements d'extraction.

Seules les solutions passant dans une aiguille ont été considérées comme injectables.

La difficulté d'aspiration du gel a été étudiée à l'aide de seringues à tuberculine, de seringues montées et d'une aiguille de fort diamètre (19 Gauges) montée sur une seringue de 10 mL en notant l'aspect du gel et en mesurant le temps d'aspiration.

En utilisant le matériel habituellement employé par les consommateurs de drogues par voie intraveineuse (eau, citron, salive, briquet, seringues, aiguilles, cuillères, dispositifs des kits stériles dispensés à cet effet et habituellement utilisés par les usagers de drogues), la dissolution complète sous forme d'un gel fluide passant au travers d'une aiguille du plus grand diamètre intérieur ne peut être obtenue qu'avec une quantité significative d'eau (supérieure à 6 mL pour 1 gélule) et des conditions particulières (casser le gel après quelques aspirations, exposer à la chaleur, injecter rapidement). Le gel fluide obtenu selon ce protocole passe dans une aiguille de faible diamètre intérieur (aiguille à tuberculine, à insuline) mais nécessite un corps de seringue de 10 mL et une poussée très progressive du piston pour éviter la séparation de l'aiguille du corps de seringue.

Selon la norme EN ISO 7886-1 (« Seringues hypodermiques stériles, non réutilisables - partie 1 : Seringue pour utilisation manuelle. Annexe G : méthode d'essai des forces nécessaires pour faire fonctionner le piston - Tableau G1 : Valeurs proposées), les limites se situent entre 0,25 et 1 daN. Les valeurs obtenues pour le contenu des gélules mises en contact avec de l'eau sont nettement supérieures à ces limites.

### Exemple 4 : Biodisponibilité du principe actif extrait d'une composition de l'exemple 2

Des essais ont été réalisés dans les conditions standard d'étude prescrites par la Pharmacopée Européenne.

La composition objet du brevet permet la libération de la totalité du principe actif dans un milieu représentatif du liquide gastrique (acide chlorhydrique 0,1 mol/L) dans un temps inférieur à 30 minutes.

La viscosité élevée de la solution obtenue, en présence d'eau, en utilisant la composition objet du brevet, ne diminue pas la biodisponibilité comme le prouve la comparaison des courbes de pharmacocinétique chez des sujets volontaires, obtenues après administration réitérée de 60 mg de chlorhydrate de méthadone sous forme :
- de deux gélules selon l'invention (1 gélule de l'exemple 2 + 1 gélule de l'exemple 2 mais renfermant 40 mg de Chlorhydrate de méthadone et d'autant moins de lactose),
- de sirop.
comme illustré à la figure 1

Une composition pharmaceutique solide destinée à la voie orale préparée selon l'invention ne peut pas être injectée par solubilisation rapide (< 2 min) dans un faible volume d'eau (1 mL) et pourtant ne diminue pas la biodisponibilité du principe actif.

Les solutions obtenues selon un protocole optimisé long et complexe, présentent des caractéristiques physiques (gel fluide opalescent d'un volume au moins supérieur à 5 mL) qui nécessiteraient, en cas d'injection de la préparation ainsi obtenue, un matériel distinct de celui proposé aujourd'hui par les programmes d'échange de seringue et les officines de pharmacie que les usagers de drogue utilisent habituellement, et enfin une compétence technique dans les différentes étapes de la manipulation, qui conduit malgré tout à une préparation liquide difficilement injectable.

### Exemple 5 : Gélification de comprimés de chlorhydrate de méthadone

### (n'illustre pas la présente invention)

On prélève la poudre contenue dans la gélule de l'Exemple 2. On la place à température ambiante dans un tube à hémolyse d'un volume de 5 ml avec 3 ml d'eau et on agite par retournement manuel du flacon à la vitesse de 50 retournements à 180° du flacon, par minute.

Une gélification intervient en moins de 30 secondes.

## Revendications

1. Une composition pharmaceutique solide sous forme de gélule contenant au moins un principe actif stupéfiant ou psychotrope ainsi qu'au moins un agent gélifiant choisi parmi l'hydroxyéthylcellulose, l'hydroxypropylcellulose, l'hydroxypropyl méthylcellulose, la méthylcellulose, l'éthyl méthylcellulose, la carboxyméthylcellulose sodique (CMC) et la carboxyméthylcellulose calcique et au moins un agent délitant dont le carboxyméthylamidon sodique, l'agent gélifiant et l'agent délitant étant pondéralement présents dans un rapport de 0,40 à 0,60 / 1 et la teneur pondérale en agent(s) gélifiant(s) étant de 10 à 20 % de la composition.

2. Une composition selon la revendication 1, **caractérisée en ce que** l'agent gélifiant est choisi parmi la carboxyméthylcellulose sodique (CMC) et la carboxyméthylcellulose calcique.

3. Une composition selon l'une des revendications 1 et 2, **caractérisée en ce qu'**il que la teneur pondérale en agent(s) gélifiant(s) est inférieure à 15 % en poids de la composition totale.

4. Une composition selon l'une des revendications 1 à 3, **caractérisée en ce qu'**il que la teneur pondérale en agent(s) délitant (s) est de 20 à 30 % de la composition totale.

5. Une composition selon l'une des revendications 1 à 4, **caractérisée en ce que** le principe actif est une substance stupéfiante utilisée en médecine telle que le lévacétylmétadol, la buprénorphine, l'hydromorphone ou la méthadone.

6. Une composition selon l'une des revendications 1 à 5, **caractérisée en ce qu'**elle comprend au moins un agent diluant et que 90 % des particules des agents diluant, gélifiant et délitant ont une taille inférieure à environ 130 µm et 50 % des particules une taille supérieure à environ 50 µm et 90 % des particules du principe actif ont une taille inférieure à 380 µm avec une médiane de distribution située aux environs de 180 µm.

7. Une composition selon l'une des revendications 1 à 6, **caractérisée en ce que** la teneur en agent gélifiant et en agent délitant est dosée pour permettre une gélification de la poudre de la composition pharmaceutique solide après addition d'une solution aqueuse en moins de 1 minute après début d'agitation du mélange de la poudre et de la solution aqueuse.

8. Un procédé de préparation d'une composition pharmaceutique solide telle que définie à l'une des revendications 1 à 7 **caractérisé en ce que** l'on mélange les différents constituants et les place dans une gélule pour obtenir la composition attendue.

## Patentansprüche

1. Eine feste pharmazeutische Zusammensetzung in Kapselform, enthaltend mindestens ein Wirkstoff, der ein Betäubungsmittel oder ein Psychopharmakon ist, sowie mindestens ein Geliermittel, das ausgewählt ist aus Hydroxethylcellulose, Hydroxypropylcellulose, Hydroxypropylmethylcellulose, Methylcellulose, Ethylmethylcellulose, Natriumcarboxymethyl-cellulose (CMC) und Calciumcarboxymethylcellulose und mindestens ein Sprengmittel, wobei Natriumcarboxymethylstärke, Geliermittel und Sprengmittel in einem Gewichtsverhältnis von 0,40 bis 0,60 / 1 vorliegen und der Gewichtsanteil des oder der Geliermittel(s) 10 bis 20 % der Zusammensetzung beträgt.

2. Eine Zusammensetzung nach Anspruch 1, **dadurch gekennzeichnet, dass** das Geliermittel ausgewählt ist aus Natriumcarboxymethylcellulose (CMC) und Calciumcarboxymethylcellulose.

3. Eine Zusammensetzung nach einem der Ansprüche 1 und 2, **dadurch gekennzeichnet, dass** der Gewichtsanteil des oder der Geliermittel(s) weniger als 15 Gew.-% der Gesamtzusammensetzung beträgt.

4. Eine Zusammensetzung nach einem der Ansprüche 1 bis 3, **dadurch gekennzeichnet, dass** der Gewichtsanteil des oder der Sprengmittel(s) 20 bis 30 % der Gesamtzusammensetzung beträgt.

5. Eine Zusammensetzung nach einem der Ansprüche 1 bis 4, **dadurch gekennzeichnet, dass** der Wirkstoff ein in der Medizin verwendetes Betäubungsmittel wie Levacetylmethadol, Buprenorphin, Hydromorphon oder Methadon ist.

6. Eine Zusammensetzung nach einem der Ansprüche 1 bis 5, **dadurch gekennzeichnet, dass** sie mindestens ein Verdünnungsmittel enthält und wobei 90 % der Teilchen von Verdünnungsmittel, Geliermittel und Sprengmittel eine Größe von weniger als ca. 130 µm haben und 50 % der Teilchen eine Größe von mehr als 50 µm haben und wobei 90 % der Teilchen des Wirkstoffs eine Größe von weniger als 380 µm haben und deren Medianwert der Verteilung bei ca. 180 µm liegt.

7. Eine Zusammensetzung nach einem der Ansprüche 1 bis 6, **dadurch gekennzeichnet, dass** der Gehalt an Geliermittel und Sprengmittel so dosiert ist, dass eine Gelierung des Pulvers der festen pharmazeutischen Zusammensetzung, nach Zugabe einer wässrigen Lösung, in weniger als 1 Minute nach Beginn der Vermischung des Pulvers mit der wässrigen Lösung möglich ist.

8. Ein Herstellungsverfahren einer festen pharmazeutischen Zusammensetzung, nach einem der Ansprüche 1-7, **dadurch gekennzeichnet dass**, die verschiedenen Bestandteile vermischt und in einer Kapsel platziert werden um die erwartete Zusammensetzung zu erhalten.

## Claims

1. A solid pharmaceutical composition in the form of a gel capsule containing at least one narcotic or psychotropic active ingredient as well as at least one gelling agent chosen from hydroxyethyl cellulose, hydroxypropyl cellulose, hydroxypropylmethyl cellulose, methyl cellulose, ethyl methyl cellulose, sodium carboxymethyl cellulose (CMC) and calcium carboxymethyl cellulose and at least one disintegrating agent of which sodium carboxymethyl starch, the gelling agent and the disintegrating agent being present in a ratio by weight of 0.40 to 0.60/1 and the content by weight of gelling agent(s) being from 10% to 20% of the composition.

2. A composition according to claim 1, **characterised in that** the gelling agent is chosen from sodium carboxymethyl cellulose (CMC) and calcium carboxymethyl cellulose.

3. A composition according to one of claims 1 and 2, **characterised in that** the content by weight of gelling agent(s) is less than 15% by weight of the total composition.

4. A composition according to one of claims 1 to 3, **characterised in that** the content by weight of disintegrating agent(s) is from 20 to 30% of the total composition.

5. A composition according to one of claims 1 to 4, **characterised in that** the active ingredient is a narcotic substance used in medicine such as levacetylmethadol, buprenorphine, hydromorphone or methadone.

6. A composition according to one of claims 1 to 5, **characterised in that** it comprises at least one diluting and that 90% of the particles of the diluting, gelling and disintegrating agents have a size less than about 130 µm and 50% of the particles a size greater than about 50 µm and 90% of the particles of the active ingredient have a size less than 380 µm with a median of the distribution located at about 180 µm.

7. A composition according to one of claims 1 to 6, **characterised in that** the content in gelling agent and in disintegrating agent is dosed in order to allow for a gelling of the powder of the solid pharmaceutical composition after the adding of an aqueous solution in less than 1 minute after the beginning of agitation of the mixture of the powder and of the aqueous solution.

8. A method for preparing a solid pharmaceutical composition such as defined in one of claims 1 to 7 **characterised in that** the various constituents are mixed and are placed in a gel capsule in order to obtain the expected composition.
